# EUROPEAN PATENT SPECIFICATION

(11) **EP 3 866 823 B1**
(45) Date of publication and mention of the grant of the patent: **26.11.2025**
(21) Application number: 19873481.6
(22) Date of filing: 18.10.2019
(51) Int. Cl.: C07C 37/70, C07C 39/23, C07D 311/80, A61K 36/00, A61K 31/05, A61K 31/352, A61K 36/185, B01D 11/02

(54) **POWDERED BOTANICAL EXTRACT PREPARATIONS AND FORMULATIONS THEREOF**
PULVERFÖRMIGE PFLANZENEXTRAKTZUBEREITUNGEN UND FORMULIERUNGEN DAVON
PRÉPARATIONS D'EXTRAITS BOTANIQUES EN POUDRE ET LEURS FORMULATIONS

(30) Priority: 19.10.2018 US 201862748266 P
(43) Date of publication of application: 25.08.2021
(73) Proprietor: Fong, Sonny, Pointe-Claire, QC H9S 4H2 (CA)
(72) Inventor: CASTILLO-GUIMOND, Diego, Laval, Québec H7N 2N6 (CA); FONG, Sonny, Québec H9S 4H2 (CA)
(74) Representative: Studio Torta S.p.A.
(86) International application number: PCT/CA2019/051477
(87) International publication number: WO 2020/077462

(56) References cited:
- WO-A1-2014/167530
- CN-A- 104 489 699
- US-A- 4 568 547
- US-A1- 2017 348 277
- US-A1- 2018 369 191

## Description

### FIELD OF THE INVENTION

The present invention pertains to the field of botanical extracts, and in particular to formulations comprising a powdered cannabis extract preparation.

### BACKGROUND

Botanical materials have long been known to be a source of medicinal substances, from traditional medicines used, for example, in traditional Chinese or Ayurvedic medicinal practice, to compounds that can be encountered on a daily basis, for example, caffeine, menthol and nicotine, to compounds that have been discovered through modern drug discovery processes, such as the anti-cancer medicines taxol and vinblastine.

The botanical materials may be used directly as herbal medicines. Alternatively, the botanical materials may undergo a treatment process to provide access to the medicinal substance. Such processes can include any combination of extraction, distillation, fractionation, concentration, fermentation, or other chemical or biological methods, and the resulting medicinal products can be in the form of extracts, decoctions, tinctures, essential oils and others.

Cannabinoids are compounds derived from cannabis plants, including, for example, *Cannabis sativa,* an annual plant in the Cannabaceae family. The plant contains about 60 cannabinoids, including tetrahydrocannabinol (THC) and cannabidiol (CBD), which can be used for the treatment of a wide range of medical conditions, including glaucoma, AIDS wasting, neuropathic pain, treatment of spasticity associated with multiple sclerosis, fibromyalgia and chemotherapy-induced nausea. Additionally, these compounds have been reported to exhibit a therapeutic effect in the treatment of allergies, autoimmune disorders and inflammation, infection, epilepsy, depression, migraine, bipolar disorders, anxiety disorder, drug dependency and withdrawal syndromes, anorexia, cachexia, and dermis regulation. THC is particularly effective as an anti-emetic drug and is administered to curb emesis, a common side effect accompanying the use of opioid analgesics and anesthetics, highly active anti-retroviral therapy and cancer chemotherapy.

Cannabinoids are lipophilic and potentially acid-labile compounds. Because of their hydrophobic nature, cannabinoids are poorly absorbed systemically from oral dosage forms because of the poor dissolution of cannabinoids in the aqueous environment of gastrointestinal tract. Oral formulations of cannabinoids, therefore, exhibit low bioavailability.

WO 2014/167530 discloses cannabinoid derivatives useful in treating pain, nausea and/or emesis, stimulating appetite, inducing a euphoric effect, inducing relaxation, and inducing a positive mood change, including formulations employing methylsulfonylmethane as a carrier.

U.S. Patent No. 4,568,547 discloses the use of methylsulfonylmethane as a binder and diluent that can impart stability and prolonged shelf life to pharmaceutical compositions containing a water sensitive pharmaceutically active agent.

An optimal oral dosage form of cannabinoids is not yet available due to the substantial 'first pass' metabolic effect which limits the oral bioavailability of cannabinoids to 6% [Karschner et al., Plasma Cannabinoid Pharmacokinetics Following Controlled Oral Δ9-Tetrahydrocannabinol and Oromucosal Cannabis Extract Administration. Clin Chem 2011 January; 57(1): 66-75]. Currently, medical cannabis is administered primarily through smoking or, alternatively, consumed orally in the form of oil, cookies, chocolates, etc. The majority of these products are not standardized, i.e. cannabinoids compositions and doses are uncontrolled, and demonstrate major disadvantages of poor bioavailability resulting in administration of very high cannabinoid doses and increased side effects, high dosing frequency, high variability, low patient compliance, and short product self-life due to formulation instability.

Although cannabis extracts are a convenient source of cannabinoids, due to the oily, tar-like nature of the extracts, they are difficult to formulate into standardized dosage forms without the use of multistep processes typically employing large volumes of organic solvents and specialized equipment.

Therefore there is a need for processes for preparing cannabis extract preparations that are easy to handle and formulate into convenient and standardized dosage forms, and that does not require large volumes of organic solvents or specialized equipment. There is also a need for a cannabis extract preparation that has the additional advantage of increased cannabinoid bioavailability.

This background information is provided to reveal information believed by the applicant to be of possible relevance to the present invention. No admission is necessarily intended, nor should be construed, that any of the preceding information constitutes prior art against the present invention.

### SUMMARY OF THE INVENTION

In accordance with an aspect of the present invention, there is provided a process for preparing a powdered cannabis extract preparation according to claim 1. Moreover, according to a second aspect of the present invention there are provided powdered cannabis extract preparations according to claim 3, a dosage form according to claim 4 and a topical formulation according to claim 10.

In accordance with another aspect of the present invention, there is provided a powdered cannabis extract preparation comprising an evenly distributed dispersion of a cannabis extract in a methylsulfonylmethane matrix.

In accordance with another aspect of the present invention, there is provided an oral dosage form comprising a preparation in accordance with the present invention, and one or more pharmaceutically acceptable excipients or carriers.

In accordance with another aspect of the present invention, there is provided a topical formulation comprising a preparation in accordance with the present invention, and one or more pharmaceutically acceptable excipients or carriers.

### BRIEF DESCRIPTION OF THE FIGURES

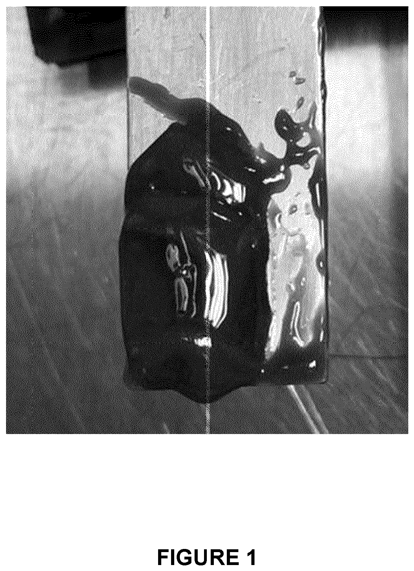

Figure 1 is a photograph of a cannabis extract prior to undergoing a process in accordance with the present invention.

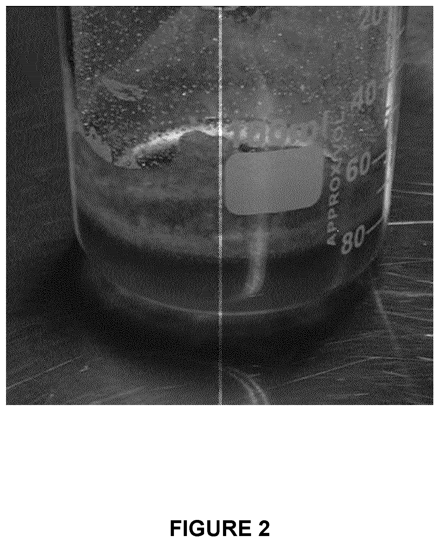

Figure 2 is a photograph of a mixture of a cannabis extract and MSM prior to solidification in a process in accordance with the present invention.

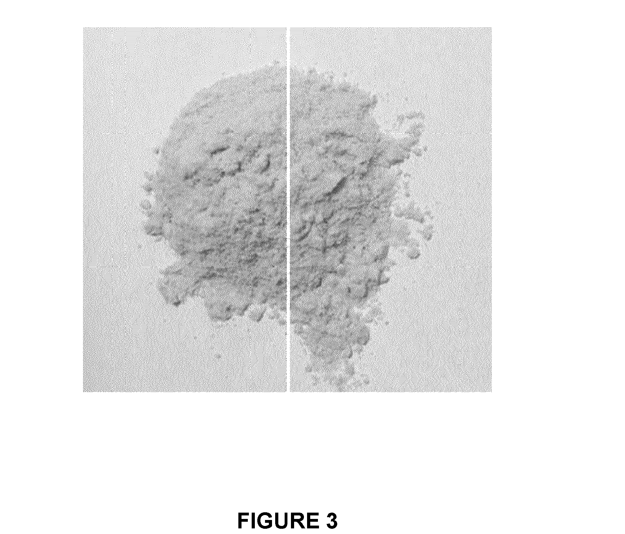

Figure 3 is a photograph of the powdered cannabis extract preparation after undergoing a process in accordance with the present invention.

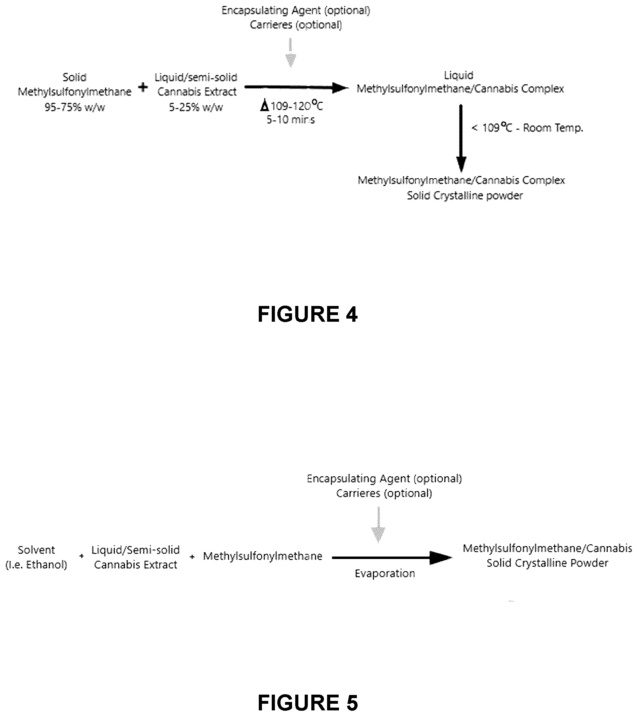

Figure 4 is a schematic depiction of a process in accordance with one embodiment of the present invention.
Figure 5 is a schematic depiction of a process in accordance with one embodiment of the present invention.

### DETAILED DESCRIPTION OF THE INVENTION

As used herein, the term "about" refers to a +/-10% variation from the nominal value. It is to be understood that such a variation is always included in a given value provided herein, whether or not it is specifically referred to.

The terms "botanicals" and "botanical material" as employed herein refer to materials including plant materials (including but not limited to leaves, stems, buds, bark, roots, and flowers of herbaceous and nonherbaceous plants), algae, macroscopic fungi, and combinations thereof, in accordance with the use of these terms in the document Botanical Drug Development, Guidance for Industry, December 2016, US Department of Health and Human Services, Food and Drug Administration Centre for Drug Evaluation and Research.

The term "Cannabis plant(s)" encompasses wild type *Cannabis sativa* and also variants thereof, including those which naturally contain different amounts of the individual cannabinoids, *Cannabis sativa* subspecies indica including the variants var. indica and var. kafiristanica, *Cannabis indica,* as well as plants which are the result of genetic crosses, self-crosses or hybrids thereof.

The term "Cannabis plant material" is to be interpreted accordingly as encompassing plant material derived from one or more cannabis plants, including leaves, buds, flowers, and stems.

In the context of this application, the terms "cannabis extract" or "extract from a cannabis plant", which are used interchangeably, encompass extracts of Cannabis plant material obtained by one or more of the following processes: pulverisation, decoction, expression, maceration, percolation, aqueous extraction, extraction with solvents such as C₁ to C₅ alcohols (*e*.*g*., ethanol), Norflurane (HFA134a), HFA227 and liquid carbon dioxide under pressure, or other similar processes. Cannabis extracts can include primary extracts prepared by such processes, which may be further purified for example by supercritical or subcritical extraction, vaporisation and chromatography. When solvents such as those listed above are used, the resultant extract may contain non-specific lipid-soluble material, which can optionally be removed by a variety of processes including "winterisation", which involves chilling to -20°C, followed by filtration to remove waxy ballast, extraction with liquid carbon dioxide and by distillation.

Preferred "cannabis extracts" include those which are obtainable by using any of the methods or processes as are known in the art for preparing extracts from cannabis plant material. The extracts are preferably substantially free of waxes and other non-specific lipid soluble material but preferably contain substantially all of the cannabinoids naturally present in the plant, most preferably in substantially the same ratios in which they occur in the intact cannabis plant.

Methylsulfonylmethane, or MSM, has been given a "GRAS" designation (Generally Recognized As Safe), and has been approved for use in pharmaceuticals, food, and cosmetics. It is also referred to as dimethyl sulfone or methyl sulfone.

The term "matrix" or "cannabis extract/MSM matrix", as used in the present specification, refers to an evenly distributed solution of a cannabis extract in a solid MSM solvent.

Unless defined otherwise, all technical and scientific terms used herein have the same meaning as commonly understood by one of ordinary skill in the art to which this invention belongs.

The present invention provides a cannabis extract preparation comprising the combination of a cannabis extract in a methylsulfonylmethane (MSM) matrix, wherein the preparation is in a powder form that is easy to handle and formulate into convenient and standardized dosage forms.

Without intending to be bound by theory, the powdered cannabis extract preparation of the present invention can be understood to be an evenly distributed dispersion of the cannabis extract in a MSM matrix. In such an embodiment, the combination of the cannabis extract and MSM further provides a composition that has the additional advantage of increased cannabinoid bioavailability upon administration to a subject relative to formulations that do not contain the MSM matrix.

In one embodiment, the composition comprises about 75% to about 95% by weight of MSM and about 5% to about 25% by weight of the cannabis extract.

In one embodiment, the powdered cannabis extract preparation consists essentially of the cannabis extract in a MSM matrix.

In certain embodiments, the powdered cannabis extract/MSM matrix may optionally be combined with one or more inactive, neutral compounds/ingredients which can be pharmaceutically acceptable excipients or carriers, including, but not limited to, binders, antioxidants, adjuvants, synergists and/or preservatives.

In certain embodiments, the cannabis extract preparations can further include encapsulating agents known in the art (*i*.*e*., phospholipids, cyclodextrins, etc.) to encapsulate the actives. These encapsulating agents can be employed with or without the use of aqueous or organic solvents.

In certain embodiments, the powdered cannabis extract/MSM preparations may optionally be combined with one or more additional pharmaceutically active components.

The processes of the present invention are particularly suitable for preparing a powdered cannabis extract preparation that can be handled and manipulated more easily than a cannabis extract that has not been processed in accordance with the present invention, which is typically in the form of an oily, tar-like substance that is difficult to manipulate, transfer and measure. The present invention therefore also provides formulation processes that offer convenient routes to the cannabis extract preparations of the present invention.

In accordance with one embodiment of the invention, there is provided a process for preparing the powdered cannabis extract preparation that employs the use of molten MSM as a solvent for the cannabis extract. In this embodiment, the process involves adding the cannabis extract to a molten MSM phase with stirring to provide a cannabis extract/MSM mixture, and stirring the resulting mixture until a single phase cannabis extract/MSM solution is formed. The cannabis extract/MSM single phase solution is allowed to cool until the mixture is solidified and the resulting solid mass is milled to provide the powdered cannabis extract preparation, wherein the powdered cannabis extract preparation comprises an evenly distributed dispersion of the cannabis extract in a MSM matrix.

In accordance with an embodiment of the disclosure, there is provided a process for preparing the powdered botanical extract preparation that employs the use of solvents to initially solubilize the botanical extract. In this embodiment, the process involves pre-dissolving the botanical extract in a suitable solvent to provide a solution of the botanical extract. MSM is added to the botanical extract solution to provide a botanical extract/MSM/solvent mixture, which is stirred until a single phase botanical extract/MSM solution is formed. The solvent is subsequently removed using any suitable solvent removal processes, until a solid botanical extract/MSM residue is formed. The resulting solid botanical extract/MSM residue is milled to provide the powdered botanical extract preparation, wherein the powdered botanical extract preparation comprises an evenly distributed dispersion of the botanical extract in a MSM matrix. In a preferred embodiment, the botanical extract is a cannabis extract.

In one embodiment, the weight ratio of botanical extract to solvent is from about 1:1 to about 1:4.

In one embodiment, the solvent used to pre-dissolve the botanical extract is a lower alcohol. In a preferred embodiment, the solvent is ethanol.

The processes of the present invention are simple, fast, efficient, and cost-effective, and can be performed using widely available standard commercial equipment. These processes are also easily scalable, and do not require the use of large volumes of organic solvents.

In those embodiments where the powdered cannabis extract/MSM preparation is optionally combined with one or more inactive, neutral ingredients or one or more additional pharmaceutically active components, the optional ingredients may be added during the process of preparing the powdered preparation, for example, while the mixture is still in its liquid form and before the solid product has been obtained.

Alternatively, the optional ingredients may be added to the powdered preparation after the process of preparing the powdered preparation has been completed, and before further formulation into suitable dosage forms.

In accordance with the present invention, the powdered cannabis extract/MSM preparation can be further incorporated into different dosage forms in accordance with formulation processes as are known in the art.

Some embodiments of the invention are directed to dosage forms that are formulated as solid articles suitable for sublingual or oral administration, such as troches, lozenges, pills, oral dissolving strips, caps or boluses. These solid dosage forms typically comprise additional excipients. They can be prepared by first mixing the powdered cannabis extract/MSM preparation of the present invention with one or more suitable excipients followed by molding or compressing the blended mixture. Both hard and chewable lozenges and troches are within the scope of the invention. In one embodiment, the oral dosage form is formulated as capsules in which the powdered cannabis extract/MSM preparation is encapsulated in soft or hard gelatin capsules.

The term "carrier" refers to a substance that serves as a vehicle for improving the efficiency of delivery and the effectiveness of a pharmaceutical composition.

The term "excipient" refers to a pharmacologically inactive substance that is formulated in combination with a pharmacologically active ingredient of a pharmaceutical composition and is inclusive of, but not limited to, disintegrants, lubricants, flavorings, bulking agents, binders, fillers, diluents, preservatives, antioxidants, and adjuvants, synergists and products used for facilitating drug absorption or solubility or for other pharmacokinetic considerations.

The term "binder" refers to a substance or compound that promotes, provides or improves cohesion, i.e., a substance that causes the components of a mixture to cohere to form a solid item that possesses integrity.

Some embodiments of the invention are directed to dosage forms that are formulated as topical formulations, including but not limited to creams, ointments and gel, using formulation methods as are known in the art. In one embodiment, the topical formulation is a transdermal patch, using formulation methods and technologies as are known in the art.

Some embodiments of the invention are directed to dosage forms that are formulated as solid articles suitable for administration as vaginal ovules or rectal suppositories, using formulation methods as are known in the art.

Some embodiments of the invention are directed to dosage forms that are formulated as liquids, including but not limited to emulsions, liposomes, dispersions, oils, and tinctures, using formulation methods as are known in the art.

Some embodiments of the invention are directed to dosage forms that are formulated as beverages and edibles, wherein the powdered cannabis extract/MSM preparation is incorporated into food and drink products.

Some embodiments of the invention are directed to dosage forms that are formulated as smokeable or vaporizable formulations.

Typical binders that can be used for formulating dosage forms mentioned above include, without limitation, natural, synthetic, or semi-synthetic glycerides, hydrogenated coco-glycerides, mineral oil, cellulose, methyl cellulose and other cellulose derivatives, gelatins, starches, sucrose and sucrose derivatives, lactose and lactose derivatives, silicones (e.g., dimethicone, bis-vinyl dimethicone/dimethicone copolymer), isopropyl myristate, isostearyl palmitate, polyvinylpyrrolidone and derivatives, and polyglycols, e.g., polyethylene glycols (PEGs), polyethylene oxides (POE), methoxypolyethylene glycols, polypropylene glycols, polybutylene glycols or derivatives thereof having a molecular weight that is sufficient to provide the necessary hardness and time for dissolution of the dosage form; for example, the acceptable molecular weight can be within the range of between about 1,000 Daltons and about 8,000 Daltons. In some embodiments, PEG-1450 or PEG-400 can be used. Non-limiting examples of some specific polyglycol derivatives that can be used are: (a) PEG-laureates and dilaureates (e.g., PEG-10-, PEG-12-, PEG-20-, PEG-32-laurates,
PEG-20- and PEG-32-dilaurates, PEG-20-glyceryl-, PEG-30-glyceryl- and PEG-40-glyceryl-laurates, PEG-80-sorbitan laurate); (b) PEG-oleates, dioleates and trioleates (e.g., PEG-12-, PEG-15-, PEG-20-, PEG-32, PEG-200- and PEG-400-oleates, PEG-20- and PEG-32-dioleates, PEG-20-trioleate, PEG-25-glyceryl trioleate, PEG-20-glyceryl- and PEG-30-glyceryl-oleates, PEG-40-sorbitan oleate); (c) PEG-stearates and distearates (e.g., PEG-15-, PEG-40-, PEG-100-stearates, PEG-32-distearate and PEG-20-glyceryl stearate) (d) castor, palm kernel, corn and soya oil derivatives of PEG (e.g., PEG-35-, PEG-40- and PEG-60-castor oils, PEG-40-, PEG-50- and PEG-60-hydrogenated castor oils, PEG-40-palm kernel oil, PEG-60-corn oil, PEG-30-soya sterol); (e) other PEG derivatives (e.g., PEG-24- and PEG-30-cholesterol, PEG-25-phytosterol, PEG-6- and PEG-8-caprate/caprylate glycerides, tocopheryl PEG-100 succinate, PEG-15-100 octylphenol products and PEG-10-100 nonylphenol products); (f) other products such as polyglyceryl-10-laurate, POE-9- and POE-23-lauryl ethers, POE-10- and POE-20-oleyl ethers, POE-20-stearyl ether, polysorbate-20 (Tween 20), polysorbate-80 (Tween 80), polyglyceryl-10-oleate, Tween 40, Tween 60, sucrose monostearate, monolaurate and monopalmitate and various products of Poloxamer series.

The dosage forms of the present invention can be formulated, as appropriate, to include disintegrants, including but not limited to starch, cellulose derivatives and alginates, crosslinked sodium carboxymethyl cellulose (corscarmellose sodium), hydroxypropylmethyl cellulose (HPMC), and crosslinked polyvinylpyrrolidone (crospovidone).

The dosage forms of the present invention can be formulated, as appropriate, to include glidants, including but not limited to silicon dioxide, colloidal anhydrous silicon and other silica compounds, and/or and or lubricants including stearic acid and salts thereof, such as magnesium stearate.

Typical excipients that can be used for formulating dosage forms mentioned above include, without limitation, gelatin, sodium saccharin, mannitol, stevioside, peppermint oil, cherry flavor, lemon oil and raspberry flavor.

As stated above, the dosage forms may optionally be formulated to further comprise one or several antioxidants. If antioxidants are used, non-limiting examples of those that can be used include α-tocopherol acetate, acetone sodium bisulfite, acetylcysteine, ascorbic acid, ascorbyl palmitate, butylated hydroxyanisole, butylated hydroxytoluene, cysteine, cysteine hydrochloride, tocopherol natural, tocopherol synthetic, dithiothreitol, monothioglycerol, nordihydroguaiaretic acid, propyl gallate, sodium bisulfite, sodium formaldehyde sulfoxylate, sodium metabisulfite, sodium sulfite, sodium thiosulfate, thiourea and tocopherols.

As stated above, the dosage forms may optionally be formulated to further comprise one or several adjuvants or synergists. If adjuvants or synergists are used, non-limiting examples of those that can be used include citric acid, EDTA (ethylenediaminetetraacetate) and salts, hydroxyquinoline sulfate, phosphoric acid and tartaric acid.

As stated above, the dosage forms may optionally further comprise one or several preservatives. If preservatives are used, non-limiting examples of those that can be used include benzalkonium chloride, benzethonium chloride, benzoic acid and salts, benzyl alcohol, boric acid and salts, cetylpyridinium chloride, cetyltrimethyl ammonium bromide, chlorobutanol, chlorocresol, chorhexidine gluconate or chlorhexidine acetate, cresol, ethanol, imidazolidinyl urea, metacresol, methylparaben, nitromersol, o-phenyl phenol, parabens, phenol, phenylmercuric acetate/nitrate, propylparaben, sodium benzoate, sorbic acids and salts, o-phenylethyl alcohol and thimerosal.

In one embodiment, the amount of inactive ingredients included in a given dosage form can be between about 10% and about 99% by weight of the final dosage form. In one embodiment, the amount of inactive ingredients included in a given dosage form can be between about 20% and about 90% by weight of the final dosage form. In one embodiment, the amount of inactive ingredients included in a given dosage form can be between about 30% and about 70% by weight of the final dosage form. In one embodiment, the amount of inactive ingredients included in a given dosage form can be between about 40% and about 60% by weight of the final dosage form. In one embodiment, the amount of inactive ingredients included in a given dosage form can be between about 75% and about 98% by weight of the final dosage form.

The invention will now be described with reference to specific examples. It will be understood that the following examples are intended to describe embodiments of the invention and are not intended to limit the invention in any way.

### EXAMPLES

### EXAMPLE 1: Molten MSM process

The following is an exemplary process for the formation of a MSM/ cannabis extract matrix.
1) In an appropriate vessel equipped with a stirrer set to medium speed, heat 18.5 g MSM at between 109-120°C until completely melted.
2) Once the MSM has melted, add 1.05 g cannabis extract.
3) Continue stirring until all of the cannabis extract has dissolved into the molten MSM.
4) Stir for an additional 5-10 minutes, and then remove from the heat source.
5) Immediately transfer liquid mixture into trays or leave in the vessel to cool to room temperature.
6) Once cooled, liquid mixture solidifies, and the solid product is milled to a powder product having desired particle size.

### EXAMPLE 2: Solvent + MSM process (outside the scope of the present invention)

The following is an exemplary process for the formation of a MSM/botanical extract matrix, wherein the botanical extract is a cannabis extract, in accordance with an embodiment of the present invention.
1) In an appropriate distillation/evaporation vessel (rotary evaporator, vacuum kettle, etc), a solution of 1.05g of the cannabis extract dissolved in 4.20g ethanol (1:4 weight ratio of extract:solvent).
2) 18.5 g MSM is added to the solution produced in step 1.
3) The vessel containing the MSM/cannabis extract solution is heated, optionally under reduced pressure, to evaporate the solvent to provide a solid residue.
4) The solid residue is collected and milled to a powder product having desired particle size.

### EXAMPLE 3: Sublingual Dosage Form

The following is an exemplary sublingual formulation prepared with the ingredients listed in Table 1. The MSM/cannabis extract matrix was prepared according to the process described in Example 1.

**Table 1:**

| **Ingredient** | **%** | **mg** |
|---|---|---|
| MSM/Cannabis Extract Matrix | 37.74 | 100 |
| Silicon Dioxide | 0.38 | 1 |
| Natural Peppermint Flavor | 0.18 | 0.5 |
| Magnesium Stearate | 1.13 | 3 |
| Stearic Acid | 1.02 | 2.7 |
| Sodium Croscarmellose | 0.38 | 1 |
| Mannitol | 59.16 | 156.8 |
| | **100.0** | **265** |

The ingredients are combined and pressed into a sublingual tablet form

### EXAMPLE 4: Placebo Dosage Form

A placebo formulation for use as a comparison prepared with the ingredients listed in Table 2.

**Table 2:**

| **Ingredient** | % | **mg** |
|---|---|---|
| Silicon Dioxide | 0.38 | 1 |
| Natural Peppermint Flavor | 0.18 | 0.5 |
| Magnesium Stearate | 1.13 | 3 |
| Stearic Acid | 1.02 | 2.7 |
| Sodium Croscarmellose | 0.38 | 1. |
| Mannitol | 96.9 | 256.8 |
| | **100.0** | **265** |

The ingredients are combined and pressed into a sublingual tablet form.

### EXAMPLE 5: Suppository Dosage Form

The following is an exemplary suppository dosage form prepared with the ingredients listed in Table 3. The MSM/cannabis extract matrix was prepared according to the process described in Example 1.

**Table 3:**

| **Ingredient** | % | **mg** |
|---|---|---|
| Semi-synthetic glycerides | 48.39 | 1354.5 |
| Hydrogenated coco-glycerides | 48 | 1315.5 |
| MSM/ Cannabis Extract Matrix | 3.61 | 100 |
| | **100** | **2770** |

The ingredients are combined and molded into a rectal suppository dosage form.

### EXAMPLE 6: Sublingual Tablet Trial

A sublingual tablet as described in Example 3, formulated to provide an effective THC dosage level of between 2.5-5.0 mg THC (the "test tablet"), was employed in a comparative crossover trial as follows. One test tablet was administered to each of 3 of our laboratory staff members against a placebo arm in a blinded fashion. Each staff member who received a test tablet noticed an onset of various effects within 5-15 minutes of administration, ranging from feeling of wellbeing, euphoria, description of a relaxed/comfortable state, pleasant "goose-bump" sensation of the upper body. The effects were reported to last approximately 1.5 to 2.5 hours in duration.

### EXAMPLE 7: Rectal Suppository Trial

A rectal suppository as described in Example 5, formulated to provide an effective THC dosage level of between 2.5-5.0 mg THC (the "test suppository"), was employed in a trial as follows. One test suppository was administered to each of 2 of our laboratory staff members. Both staff members reported onset of effects approximately 30 to 45 minutes after administration. Effects experienced ranged from sense of wellbeing, euphoria, perceived slight increase in heart rate and energy level, and clear-mindedness. Effects lasted beyond 2.5 hours.

## Claims

1. A process for preparing a powdered cannabis extract preparation, the process comprising the steps of:
i) providing a molten methylsulfonylmethane (MSM) phase,
ii) adding a cannabis extract to the molten MSM phase to form a liquid cannabis extract/MSM mixture,
iii) stirring the liquid cannabis extract/MSM mixture until the cannabis extract has dissolved in the MSM to form a single phase cannabis extract/MSM solution,
iv) cooling the single phase cannabis extract/MSM solution to form a solid cannabis extract/MSM mixture; and
v) milling the solid cannabis extract/MSM mixture to provide the powdered cannabis extract preparation,
wherein the powdered cannabis extract preparation comprises an evenly distributed dispersion of the cannabis extract in a MSM matrix.

2. The process of claim 1, further comprising a step of adding an encapsulating agent or carrier to the cannabis extract/MSM mixture prior to cooling.

3. A powdered cannabis extract preparation comprising an evenly distributed dispersion of a cannabis extract in a methylsulfonylmethane matrix prepared using the process as defined in claim 1 or 2, optionally comprising one or more pharmaceutically acceptable excipients or carriers.

4. A dosage form comprising a preparation as defined in claim 3, and one or more pharmaceutically acceptable excipients or carriers.

5. The dosage form of claim 4, wherein the dosage form is an oral dosage form selected from a troche, a lozenge, a pill, an oral dissolving strip, a tablet, a capsule, or a bolus.

6. The dosage form of claim 4, wherein the dosage form is an oral dosage form formulated as a beverage or an edible, and wherein the powdered cannabis extract/MSM composition is incorporated into a foodstuff.

7. The dosage form of claim 4, wherein the dosage form is formulated as a vaginal ovule or a rectal suppository.

8. The dosage form of claim 4, wherein the dosage form is formulated as an emulsion, a liposome, a dispersion, an oil, or a tincture.

9. The dosage form of claim 8, wherein the dosage form is a smokeable or vaporizable formulation.

10. A topical formulation comprising a preparation as defined in claim 3, and one or more pharmaceutically acceptable excipients or carriers, wherein the topical formulation is a cream, an ointment, a gel or a transdermal patch.

## Patentansprüche

1. Verfahren zur Herstellung einer pulverisierten Cannabisextraktzubereitung, wobei das Verfahren die folgenden Schritte umfasst:
i) Bereitstellen einer geschmolzenen Methylsulfonylmethan (MSM)-Phase,
ii) Zugeben eines Cannabisextrakts zu der geschmolzenen MSM-Phase, um ein flüssiges Cannabisextrakt/MSM-Gemisch zu bilden,
iii) Rühren des flüssigen Cannabisextrakt/MSM-Gemisches, bis sich der Cannabisextrakt in dem MSM gelöst hat, um eine einphasige Cannabisextrakt/MSM-Lösung zu bilden,
iv) Abkühlen der einphasigen Cannabisextrakt/MSM-Lösung, um ein feste Cannabisextrakt/MSM-Gemisch zu bilden; und
v) Mahlen des festen Cannabisextrakts/MSM-Gemisches, um die pulverisierte Cannabisextraktzubereitung bereitzustellen,
wobei die pulverisierte Cannabisextraktzubereitung eine gleichmäßig verteilte Dispersion des Cannabisextrakts in einer MSM-Matrix umfasst.

2. Verfahren nach Anspruch 1, ferner umfassend einen Schritt zum Zugeben eines Verkapselungsmittels oder Trägers zu dem Cannabisextrakt/MSM-Gemisch vor dem Abkühlen.

3. Pulverisierte Cannabisextraktzubereitung, umfassend eine gleichmäßig verteilte Dispersion von einem Cannabisextrakt in einer Methylsulfonylmethanmatrix, hergestellt unter Verwendung des Verfahrens nach Anspruch 1 oder 2, optional umfassend einen oder mehrere pharmazeutisch verträgliche Hilfsstoffe oder Träger.

4. Darreichungsform, umfassend eine Zubereitung nach Anspruch 3 und einen oder mehrere pharmazeutisch verträgliche Hilfsstoffe oder Träger.

5. Darreichungsform nach Anspruch 4, wobei die Darreichungsform eine orale Darreichungsform ist, die aus einer Troche, einer Lutschtablette, einer Pille, einem oralen Auflösungsstreifen, einer Tablette, einer Kapsel oder einem Bolus ausgewählt ist.

6. Darreichungsform nach Anspruch 4, wobei die Darreichungsform eine als Getränk oder Essbares formulierte orale Darreichungsform ist und wobei die pulverisierte Cannabisextrakt/MSM-Zusammensetzung in ein Lebensmittel eingearbeitet ist.

7. Darreichungsform nach Anspruch 4, wobei die Darreichungsform als Vaginal-Ovulum oder als Rektalzäpfchen formuliert ist.

8. Darreichungsform nach Anspruch 4, wobei die Darreichungsform als eine Emulsion, ein Liposom, eine Dispersion, ein Öl oder eine Tinktur formuliert ist.

9. Darreichungsform nach Anspruch 8, wobei die Darreichungsform eine rauchbare oder verdampfbare Formulierung ist.

10. Topische Formulierung, umfassend eine Zubereitung nach Anspruch 3 und einen oder mehrere pharmazeutisch verträgliche Hilfsstoffe oder Träger, wobei die topische Formulierung eine Creme, eine Salbe, ein Gel oder ein transdermales Pflaster ist.

## Revendications

1. Procédé de préparation d'une préparation d'extrait de cannabis en poudre, le procédé comprenant les étapes suivantes consistant à :
i) fournir une phase fondue de méthylsulfonylméthane (MSM),
ii) ajouter un extrait de cannabis à la phase MSM fondue pour former un mélange liquide d'extrait de cannabis/MSM,
iii) agiter le mélange liquide d'extrait de cannabis/MSM jusqu'à ce que l'extrait de cannabis se soit dissous dans le MSM pour former une solution monophasée d'extrait de cannabis/MSM,
iv) refroidir la solution monophasée d'extrait de cannabis/MSM pour former un mélange solide d'extrait de cannabis/MSM ; et
v) broyer le mélange solide d'extrait de cannabis/MSM pour obtenir la préparation d'extrait de cannabis en poudre,
dans lequel la préparation d'extrait de cannabis en poudre comprend une dispersion uniformément répartie de l'extrait de cannabis dans une matrice MSM.

2. Procédé selon la revendication 1, comprenant en outre une étape d'ajout d'un agent d'encapsulation ou d'un support au mélange d'extrait de cannabis/MSM avant le refroidissement.

3. Préparation d'extrait de cannabis en poudre comprenant une dispersion uniformément répartie d'un extrait de cannabis dans une matrice de méthylsulfonylméthane préparé selon le procédé défini dans la revendication 1 ou 2, comprenant éventuellement un ou plusieurs excipients ou supports pharmaceutiquement acceptables.

4. Forme galénique comprenant une préparation telle que définie dans la revendication 3, et un ou plusieurs excipients ou supports pharmaceutiquement acceptables.

5. Forme posologique selon la revendication 4, dans laquelle la forme posologique est une forme posologique orale choisie parmi une troche, une pastille, une pilule, une bande dissolvante orale, un comprimé, une capsule ou un bolus.

6. Forme posologique selon la revendication 4, dans laquelle la forme posologique est un dosage oral formulé sous forme de boisson ou de produit comestible, et dans laquelle la composition d'extrait de cannabis en poudre/MSM est incorporée dans un produit alimentaire.

7. Forme pharmaceutique selon la revendication 4, dans laquelle la forme pharmaceutique est formulée sous forme d'ovule vaginal ou de suppositoire rectal.

8. Forme posologique selon la revendication 4, dans laquelle la forme posologique est formulée sous forme d'émulsion, de liposome, de dispersion, d'huile ou de teinture.

9. Forme posologique selon la revendication 8, dans laquelle la forme posologique est une formulation fumable ou vaporisable.

10. Formulation topique comprenant une préparation telle que définie dans la revendication 3, et un ou plusieurs excipients ou supports pharmaceutiquement acceptables, dans laquelle la formulation topique est une crème, une pommade, un gel ou un patch transdermique.
